# EUROPEAN PATENT APPLICATION

(11) **EP 4 400 577 A1**
(43) Date of publication of application: **17.07.2024**
(21) Application number: 22825002.3
(22) Date of filing: 14.06.2022
(51) Int. Cl.: C12N 1/20, A23K 10/18, A23K 50/10, C12N 15/31

(54) **NOVEL BACTERIUM THAT CONTRIBUTES TO MASS PRODUCTION OF PROPIONIC ACID PRECURSOR SUBSTANCE**

(30) Priority: 18.06.2021 JP 2021101574
(71) Applicant: NATIONAL AGRICULTURE AND FOOD RESEARCH ORGANIZATION, Tsukuba-shi, Ibaraki 305-8517 (JP)
(72) Inventor: SHINKAI, Takumi, Ibaraki, 3050091 (JP)
(74) Representative: J A Kemp LLP
(86) International application number: PCT/JP2022/023811
(87) International publication number: WO 2022/265014

(57) **Abstract**

Provided is a bacterium of genus *Prevotella* having a property that proportion of malic acid and lactic acid in organic acids in fermentation products is 20% or higher for enabling enhancement of propionic acid production and reduction of methane production in the rumen of domestic ruminants.

## Description

### [Technical Field]

The present invention relates to *Prevotella lacticifex,* a novel species of bacterium belonging to genus *Prevotella,* and a feed additive and a feed using the same.

### [Background Art]

Components of feed such as grass ingested by a domestic ruminant are decomposed and fermented by a microbial group inhabiting the rumen, and converted to organic acids such as acetic acid and propionic acid. Among the organic acids, propionic acid is a key fermentation product for improving the feed efficiency of the domestic animal because propionic acid is converted to glucose in the domestic ruminant body and becomes a major source of energy for the domestic ruminant. In addition, the propionic acid production in the rumen is known to be competitive with methane (a greenhouse gas) production in the light of hydrogen use, and great expectations are placed on the enhancement of propionic acid production as a technology to reduce methane released by domestic ruminants.

Technologies for enhancing propionic acid implemented so far include increasing the use of high-energy feed such as grain feed and supplementing antimicrobial substances such as monensin (Non Patent Literature 1). Technologies for reducing methane have also been developed, such as switching to high-energy feed, reducing the number of animals associated with productivity improvement, and using chemical substances such as 3-nitrooxypropanol (3-NOP) (Non Patent Literature 2), but none of these technologies have involved probiotics which will lead to alteration of rumen microbiota.

### [Citation List]

### [Non Patent Literature]

[Non Patent Literature 1] Methanogens: methane producers of the rumen and mitigation strategies (2010) Sarah E Hook, Andre-Denis G Wright, Brian W McBride. Archaea. doi: 10.1155/2010/945785.
[Non Patent Literature 2] Effects of 3-nitrooxypropanol on rumen fermentation, lactational performance, and resumption of ovarian cyclicity in dairy cows (2020) Melgar A., M.T. Harper, J. Oh, F. Giallongo, M.E. Young, T.L. Ott, S. Duval, A.N. Hristov. Journal of Dairy Science 103(1):410-432. doi: 10.3168/jds.2019-17085.

### [Summary of Invention]

### [Technical Problem]

Grain feed supplementation is already a common usage technology, and its further increase may lead to the development of production diseases such as acidosis, so more increase in the usage amount is limited. The use of antimicrobial substances has been restricted worldwide out of concerns about the emergence of antimicrobial resistant bacteria. There are several probiotics to improve rumen fermentation and intestinal microbiota in domestic ruminants, such as CALSPORIN (Asahi Calpis Wellness Co., Ltd.), which utilizes bacteria of genus Bacillus, Bovactin (Miyarisan Pharmaceutical Co., Ltd.), and Rumisan (NISSAN GOSEI KOGYO CO., LTD.). However, all of these are probiotics intended for improvement in the balance of the microbiota to increase productivity, and are not intended for alteration to the specific fermentation to produce high propionic acid or low methane production, so they cannot be expected to enhance propionic acid production or reduce methane production.

In response to such a background, the present invention was made with an object of providing a microorganism capable of enhancing propionic acid production and reducing methane production in the rumen of domestic ruminants.

### [Solution to Problem]

As a result of intensive studies to solve the above problem, the inventor found that a bacterium isolated from cows producing high propionic acid and low methane produce a high proportion of propionic acid precursors and, among the precursors, produce malic acid and lactic acid in particularly large amount. Malic acid and lactic acid consume hydrogen to be metabolized to propionic acid, and a methane-producing bacterium also consumes hydrogen to produce methane. Therefore, it is assumed that the above bacterium, which produces large amount of malic acid and lactic acid, not only increases the amount of propionic acid production, but also inhibits methane production by a methane-producing bacterium through reduction of the amount of hydrogen.

The present invention was completed based on the above findings.

That is, the present invention provides the following [1] to [10].
[1] A bacterium of genus *Prevotella* having a 16SrDNA consisting of a nucleotide sequence having 98.7% or higher homology with a nucleotide sequence shown in SEQ ID NO:10.
[2] The bacterium of genus *Prevotella* according to [1], wherein the bacterium has a property that proportion of malic acid and lactic acid in organic acids in fermentation products is 20% or higher.
[3] The bacterium of genus *Prevotella* according to [1], wherein the bacterium has a property that proportion of succinic acid, malic acid, propionic acid and lactic acid in organic acids in fermentation products is 65% or higher.
[4] The bacterium of genus *Prevotella* according to [1], wherein the bacterium has the following properties 1) to 6) :
   1) the bacterium is a bacillus negative for Gram stain and does not form spores,
   2) the bacterium is negative for catalase test and obligately anaerobic,
   3) the bacterium generates acids when D-glucose, lactose, saccharose, maltose, salicin, L-arabinose, cellobiose, D-mannose or D-raffinose is added, but does not generate acids when D-mannitol, glycerol, D-meletitose, D-sorbitol or D-trehalose is added,
   4) a major menaquinone of the bacterium is MK11,
   5) a most major bacterial fatty acid of the bacterium is anteiso-C15:0, and
   6) a genomic DNA of the bacterium has a GC content ranging from 49.4% to 49.9%.
[5] The bacterium of genus *Prevotella* according to [1], wherein the bacterium has an ANI value of 95% or higher against a strain identified by an accession number NITE BP-03463.
[6] The bacterium of genus *Prevotella* according to [1], wherein the bacterium has a dDDH value of 70% or higher against a strain identified by an accession number NITE BP-03463.
[7] The bacterium of genus *Prevotella* according to [1], wherein the bacterium has a 16SrDNA consisting of a nucleotide sequence shown in any one of SEQ ID NO:1 to 11.
[8] The bacterium of genus *Prevotella* according to [1], wherein the bacterium is a strain identified by an accession number NITE BP-03463.
[9] A feed additive containing the bacterium of genus *Prevotella* according to any one of [1] to [8].
[10] A feed containing the bacterium of genus *Prevotella* according to any one of [1] to [8].

The present description includes part or all of the content as disclosed in the description and/or drawings of Japanese Patent Application No.2021-101574, which is a priority document of the present application.

### [Advantageous Effect of Invention]

The present invention provides *Prevotella lacticifex,* a novel species of bacterium belonging to genus *Prevotella.* This novel species of bacterium can be utilized as a feed additive or a feed for enhancing propionic acid production and reducing methane production in the rumen of domestic ruminants.

### [Brief Description of Drawing]

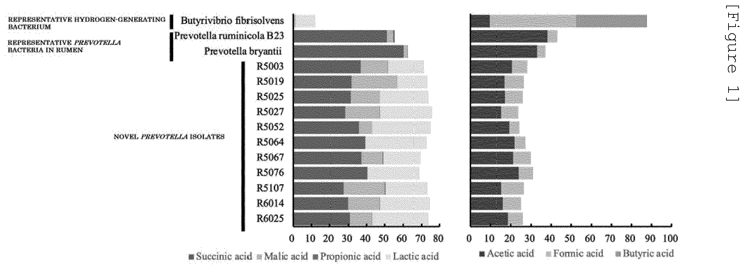

[Figure 1] Figure 1 shows the proportion of each fermentation product of novel bacterial isolates of genus *Prevotella.* The left figure shows the proportion of propionic acid and its precursors (fermentation products of the hydrogen-consuming pathway), and the right figure shows the proportion of fermentation products of the hydrogen-generating pathway, such as acetic acid.

### [Description of Embodiments]

The present invention is described in detail below.

The bacterium of genus *Prevotella* of the present invention has a 16SrDNA consisting of a nucleotide sequence having 98.7% or higher homology with a nucleotide sequence shown in SEQ ID NO:10.

The nucleotide sequence shown in SEQ ID NO:10 is a 16SrDNA nucleotide sequence of R6014, a strain of genus *Prevotella* which will be described below. R6014 is considered to be a novel species of genus *Prevotella,* and in general, a group of strains showing 16SrDNA homology of 98.7% or higher are considered as the same species. Therefore, the bacterium of genus *Prevotella* of the present invention includes a novel species of genus *Prevotella.* This novel species did not have a name at the time of filing the Japanese Patent Application No.2021-101574 (June 18, 2021), which is a priority document of the present application, but was subsequently named *Prevotella lacticifex* (Shinkai et al., Int. J. Syst. Evol. Microbiol. 2022;72:005278).

Whether or not strains are of the same species is determined by a homology value of a DNA-DNA hybridization test in addition to a 16SrDNA nucleotide sequence. In general, a group of strains with the homology value of 70% or higher in a DNA-DNA hybridization test are considered as the same species. Thus, the bacterium of genus *Prevotella* of the present invention may be a bacterium having a homology value of 70% or higher in a DNA-DNA hybridization test against the strain of genus *Prevotella* R6014.

The homology value of DNA-DNA hybridization (DDH) is obtained experimentally, but because of the variation in the value depending on the level of proficiency in the experiment and the low precision of the experiment, in recent years, Average Nucleotide Identity (ANI) analysis or digital DNA-DNA hybridization (dDDH) is more often used as an alternative method by obtaining a draft genome by DNA sequencing. In general, a group of strains with an ANI value of 95 to 96% or higher are considered as the same species, and a group of strains with a dDDH value of 70% or higher are considered as the same species. Thus, the bacterium of genus *Prevotella* of the present invention may be a bacterium having an ANI value of 95% or higher or having a dDDH value of 70% or higher against the strain of genus *Prevotella* R6014. The present inventor has performed an ANI analysis in accordance with a known literature (Rodriguez-R LM & Konstantinidis KT (2016). The enveomics collection: a toolbox for specialized analyses of microbial genomes and metagenomes. PeerJ Preprints 4:e1900v1.) and has confirmed that an ANI value between novel isolates which will be described below is 98% or higher. The inventor has also performed a dDDH analysis in accordance with a known literature (Meier-Kolthoff, J.P., Auch, A.F., Klenk, H.-P., Goker, M. Genome sequence-based species delimitation with confidence intervals and improved distance functions. BMC Bioinformatics 14:60, 2013.) and has confirmed that a dDDH value between novel isolates which will be described below is 89% or higher.

The bacterium of genus *Prevotella* of the present invention has a property that the proportion of malic acid and lactic acid in organic acids (propionic acid, succinic acid, malic acid, lactic acid, acetic acid, formic acid and butyric acid) in fermentation products is high. Specifically, the proportion of malic acid and lactic acid in organic acids is, for example, 20% or higher, 25% or higher, or 30% or higher. The high proportion of malic acid and lactic acid achieves a favorable effect of not only enhancing propionic acid production by providing precursors to propionic acid but also inhibiting methane production by methane-producing bacteria through reduction of the amount of hydrogen.

In addition, the bacterium of genus *Prevotella* of the present invention has a property that the proportion of propionic acid and its precursors (succinic acid, malic acid and lactic acid) in organic acids in fermentation products is high. Specifically, the proportion of propionic acid and its precursors in organic acids is, for example, 65% or higher, 70% or higher, or 75% or higher. The high proportion of propionic acid and its precursors achieves a favorable effect of providing precursors to propionic acid to enhance propionic acid production.

Furthermore, the bacterium of genus *Prevotella* of the present invention has the following properties 1) to 6) :
1) the bacterium is a bacillus negative for Gram stain and does not form spores,
2) the bacterium is negative for catalase test and obligately anaerobic,
3) the bacterium generates acids when D-glucose, lactose, saccharose, maltose, salicin, L-arabinose, cellobiose, D-mannose or D-raffinose is added, but does not generate acids when D-mannitol, glycerol, D-meletitose, D-sorbitol or D-trehalose is added,
4) a major menaquinone of the bacterium is MK11,
5) a most major bacterial fatty acid of the bacterium is anteiso-C15:0, and
6) a genomic DNA of the bacterium has a GC content ranging from 49.4% to 49.9%. The results of acid generating test of the above 3) is the results obtained when API 20A, a kit for biochemical identification of anaerobic bacteria (bioMerieux Japan Ltd.), was used.

The inventor has also conducted genetic analysis of novel isolates which will be described below, and has confirmed that these strains possess L-lactate dehydrogenase gene in their genome. Thus, the possession of L-lactate dehydrogenase gene in the genome is another characteristic of the bacterium of genus *Prevotella* of the present invention.

Strains included in the bacterium of genus *Prevotella* of the present invention include R5003, R5019, R5025, R5027, R5052, R5064, R5067, R5076, R5107, R6014, and R6025, which were isolated by the inventor. Among these, R6014 has been deposited internationally as described below.
1) Name and address of the International Depositary Authority
   Name: NITE Patent Microorganisms Depositary, Incorporated Administrative Agency National Institute of Technology and Evaluation
   Address: 2-5-8, Kazusakamatari, Kisarazu-shi, Chiba 2920818, Japan
2) Domestic deposit date: April 20, 2021
3) Domestic accession No.: NITE P-03463
4) Date of transfer to international deposit: May 6, 2022
5) International accession No.: NITE BP-03463

Since a strain can be identified by a 16SrDNA nucleotide sequence, a strain having a 16SrDNA consisting of the nucleotide sequence shown in SEQ ID NO:1 (16SrDNA nucleotide sequence of R5003), a strain having a 16SrDNA consisting of the nucleotide sequence shown in SEQ ID NO:2 (16SrDNA nucleotide sequence of R5019), a strain having a 16SrDNA consisting of the nucleotide sequence shown in SEQ ID NO:3 (16SrDNA nucleotide sequence of R5025), a strain having a 16SrDNA consisting of the nucleotide sequence shown in SEQ ID NO:4 (16SrDNA nucleotide sequence of R5027), a strain having a 16SrDNA consisting of the nucleotide sequence shown in SEQ ID NO:5 (16SrDNA nucleotide sequence of R5052), a strain having a 16SrDNA consisting of the nucleotide sequence shown in SEQ ID NO:6 (16SrDNA nucleotide sequence of R5064), a strain having a 16SrDNA consisting of the nucleotide sequence shown in SEQ ID NO:7 (16SrDNA nucleotide sequence of R5067), a strain having a 16SrDNA consisting of the nucleotide sequence shown in SEQ ID NO:8 (16SrDNA nucleotide sequence of R5076), a strain having a 16SrDNA consisting of the nucleotide sequence shown in SEQ ID NO:9 (16SrDNA nucleotide sequence of R5107), a strain having a 16SrDNA consisting of the nucleotide sequence shown in SEQ ID NO:10 (16SrDNA nucleotide sequence of R6014), and a strain having a 16SrDNA consisting of the nucleotide sequence shown in SEQ ID NO:11 (16SrDNA nucleotide sequence of R6025) can be considered as a strain included in the bacterium of genus *Prevotella* of the present invention.

Since the bacterium of genus *Prevotella* of the present invention has an excellent property of enhancing propionic acid production and inhibiting methane production, it can be utilized as a feed additive or a feed in order for the bacterium to consistently inhabit the digestive tract of animals.

In the same manner as known probiotics, the feed additive of the present invention can be prepared by freeze-drying the bacteria of genus *Prevotella* of the present invention and adding other ingredients as needed. The culture solution of the bacteria of genus *Prevotella* of the present invention may also be used as a feed additive without freeze-drying.

The feed of the present invention can be prepared by adding the bacteria of genus *Prevotella* of the present invention (e.g., freeze-dried bacteria, bacterial culture solution) to a general feed.

The feed additive or the feed of the present invention may be fed to any animal, but it is preferably fed to ruminants, such as dairy cows, beef cattle, sheep, and goats.

### [Examples]

The present invention will be further described in more detail below using Examples, but the invention is not limited to these Examples.

### [Example 1] Isolation and identification of strains

Two Holstein dairy cows managed by the Institute of Livestock and Grassland Science, National Agriculture and Food Research Organization were used as a source of isolation of a novel bacterium of genus *Prevotella,* characteristic of low methane-producing cows. Using a probe for collecting rumen gastric juice (Luminar, Sanshin Industrial Co., Ltd.), rumen gastric juice was orally collected, filtered through a triple layer of sterile gauze, and diluted using an anaerobic diluent (Bryant & Burkey, 1953). The diluted bacterial suspension was immediately inoculated into modified a YTR agar medium and roll tubes were prepared.

The method for preparing the YTR agar medium was in accordance with the method by Bryant & Burkey (1953). The composition of the modified YTR agar medium was as follows.

Per liter: yeast extract, 1.2 g (Oxoid); Bacto peptone, 2 g (Difco); mineral solution I, 75 mL; mineral solution II, 75 mL; bovine rumen gastric juice prepared for culture medium, 300 mL; glucose, 1.5 g; cellobiose, 1.5 g; L-cysteine hydrochloride, 3 g; 0.1% resazurin solution, 1 ml; 0.5 g/L hemin solution, 10 mL; 8% NaHCOs solution, 50 mL; bacto agar 1.2 g (Difco); distilled water, 500 mL. A hemin solution was prepared as follows: 50 mg of hemin was dissolved in 1 ml of 1N NaOH solution and diluted with 100 ml of distilled water. Mineral solutions I and II are described in Bryant and Burkey (1953).

After roll tubes were cultured at 37°C for 48 to 72 hours, the resulting colonies were extracted and inoculated into a new modified YTR agar medium. After bacterial growth, DNA was extracted and 16S rRNA gene was PCR-amplified using Takara Ex Tag HS (Takara) and primer sets 27f (5'-AGAGTTTGATCMTGGCTCAG-3') (SEQ ID NO:12) and 1492r (5'-GGGYTACCTTGTTACGACTT-3') (SEQ ID NO:13). The PCR product was subjected to electrophoresis to confirm the specific amplification, and then was purified (ExoSAP-IT, Applied Biosystems) and its nucleotide sequence was decoded (Eurofins Genomics Co., Ltd.). The obtained sequence was compared with the sequence of pyrosequencing that had been detected as genus *Prevotella* specific to low methane-producing cows and with the sequence of the clone that had been detected by clone library analysis of the full-length 16S rRNA to confirm that it was the sequence of the target strain.

The novel strains of genus *Prevotella* isolated were named R5003, R5019, R5025, R5027, R5052, R5064, R5067, R5076, R5107, R6014, and R6025, and each 16S rDNA sequence of these strains was shown in SEQ ID NOs:1, 2, 3, 4, 5, 6, 7, 8, 9, 10, and 11, respectively.

### [Example 2] Culture of strains and analysis of fermentation products

The type strains (*Prevotella ruminicola* JCM8958, P. *bryantii* DSM11371, and *Butyrivibrio fibrisolvens* JCM6563) were obtained by purchase from a Depositary Authority for strains. The type strains and the novel *Prevotella* strains isolated were inoculated in a modified YTR slant agar medium and subcultured at 37°C under anaerobic conditions.

For monitoring amplification and measuring fermentation products, a glucose medium (modified PYG medium shown in DSMZ medium 104, excluding beef extract and tween 80) was used. Strains were subcultured by inoculating 0.2 ml of bacterial growth medium in the early to mid-term logarithmic growth phase (OD₆₆₀ = 0.3 - 0.5) into 5 ml of fresh glucose medium, and pre-pre culture, pre culture, and main culture were conducted by maintaining a high level of growth activity. Bacterial growth was measured every 10 minutes by a turbidimeter (OD-Monitor C&T, TAITEC Co., Ltd.) installed in a constant temperature incubator shaker (120 rpm, Bio-Shaker BR-40LF, TAITEC Co., Ltd.). Fermentation products were analyzed by collecting a medium at the stationary phase from 44 to 48 hours after the start of culture. The culturing was repeated three times. The culture solution was purified and organic acids in the culture solution were comprehensively quantified using an organic acid analysis system by HPLC (SHIMADZU Co., Ltd.).

The proportion of organic acids in each strain culture solution is shown in Figure 1. The culture solution of the novel isolates of genus *Prevotella* contained a high proportion of propionic acid and its precursors (succinic acid, malic acid, and lactic acid) and a low proportion of fermentation products of the hydrogen-generating pathway (acetic acid, formic acid, and butyric acid). In addition, the novel isolates of genus *Prevotella* produced more amount of malic acid and lactic acid than the type strains.

### [Reference Literature]

Bryant MP & Burkey LA (1953) Cultural methods and some characteristics of some of the more numerous groups of bacteria in the bovine rumen. J Dairy Sci 36: 205-217.

All publications, patents and patent applications cited herein are incorporated herein by reference without modification.

### [Industrial Applicability]

The present invention potentially has industrial applications as a probiotic to alter the rumen fermentation in ruminants such as dairy cows, beef cattle, sheep, and goats to the propionic acid-type fermentation. It could be used in the future as a reducer of methane resulting from bovine gastric fermentation.

## Claims

1. A bacterium of genus *Prevotella* having a 16SrDNA consisting of a nucleotide sequence having 98.7% or higher homology with a nucleotide sequence shown in SEQ ID NO:10.

2. The bacterium of genus *Prevotella* according to claim 1, wherein the bacterium has a property that proportion of malic acid and lactic acid in organic acids in fermentation products is 20% or higher.

3. The bacterium of genus *Prevotella* according to claim 1, wherein the bacterium has a property that proportion of succinic acid, malic acid, propionic acid and lactic acid in organic acids in fermentation products is 65% or higher.

4. The bacterium of genus *Prevotella* according to claim 1, wherein the bacterium has the following properties 1) to 6) :
1) the bacterium is a bacillus negative for Gram stain and does not form spores,
2) the bacterium is negative for catalase test and obligately anaerobic,
3) the bacterium generates acids when D-glucose, lactose, saccharose, maltose, salicin, L-arabinose, cellobiose, D-mannose or D-raffinose is added, but does not generate acids when D-mannitol, glycerol, D-meletitose, D-sorbitol or D-trehalose is added,
4) a major menaquinone of the bacterium is MK11,
5) a most major bacterial fatty acid of the bacterium is anteiso-C15:0, and
6) a genomic DNA of the bacterium has a GC content ranging from 49.4% to 49.9%.

5. The bacterium of genus *Prevotella* according to claim 1, wherein the bacterium has an ANI value of 95% or higher against a strain identified by an accession number NITE BP-03463.

6. The bacterium of genus *Prevotella* according to claim 1, wherein the bacterium has a dDDH value of 70% or higher against a strain identified by an accession number NITE BP-03463.

7. The bacterium of genus *Prevotella* according to claim 1, wherein the bacterium has a 16SrDNA consisting of a nucleotide sequence shown in any one of SEQ ID NO:1 to 11.

8. The bacterium of genus *Prevotella* according to claim 1, wherein the bacterium is a strain identified by an accession number NITE BP-03463.

9. A feed additive containing the bacterium of genus *Prevotella* according to any one of claims 1 to 8.

10. A feed containing the bacterium of genus *Prevotella* according to any one of claims 1 to 8.
